# EUROPÄISCHE PATENTSCHRIFT

(11) **EP 0 608 512 B1**
(45) Veröffentlichungstag und Bekanntmachung des Hinweises auf die Patenterteilung: **26.03.1997**
(21) Anmeldenummer: 93119302.3
(22) Anmeldetag: 30.11.1993
(51) Int. Cl.: A61F 13/46, B32B 27/12

(54) **Decklage zur Verwendung bei körperflüssigkeitsabsorbierenden Wegwerfartikeln**
Top sheet for use in body fluids absorbing disposable articles
Couche de surface pour articles d'hygiene absorbants jetables

(30) Priorität: 30.11.1992 JP 320619/92
(43) Veröffentlichungstag der Anmeldung: 03.08.1994
(73) Patentinhaber: MITSUI PETROCHEMICAL INDUSTRIES, LTD., Tokyo (JP); UNI-CHARM CORPORATION, Kawanoe-shi Ehime-ken (JP)
(72) Erfinder: Takamitsu, Igaue, Ehime-ken (JP); Tsutomu, Kido, Ehime-ken (JP); Hisashi, Takai, Ehime-ken (JP)
(74) Vertreter: Sperling, Rüdiger, Dipl.-Ing.

(56) Entgegenhaltungen:
- EP-A- 0 336 578
- EP-A- 0 358 031
- DE-A- 3 517 640
- PATENT ABSTRACTS OF JAPAN, unexamined applications, C Sektion, Band 16, Nr. 437, 11. September 1992, THE PATENT OFFICE JAPANESE GOVERNMENT Seite 110 C 984; & JP-A-4-152 945 (UNI CHARM CORP.)
- PATENT ABSTRACTS OF JAPAN, unexamined applications, C Sektion, Band 16, Nr. 466, 28. September 1992, THE PATENT OFFICE JAPANESE GOVERNMENT Seite 115 C 989; & JP-A-4-166 151 (UNI CHARM CORP.)
- PATENT ABSTRACTS OF JAPAN, unexamined applications, C Sektion, Band 16, Nr. 114, 23. MUrz 1992, THE PATENT OFFICE JAPANESE GOVERNMENT Seite 99 C 921; & JP-A-3-286 762 (UNI CHARM CORP.)

## Beschreibung

### HINTERGRUND DER ERFINDUNG

Die vorliegende Erfindung betrifft flüssigkeitsdurchlässige Decklagen zur Verwendung bei körperflüssigkeitsabsorbierenden Wegwerfartikeln, wie bei Monatsbinden, Wegwerfwindeln und Trainingshosen.

Ein recht bekanntes Verfahren zur Herstellung von bei körperflüssigkeitsabsorbierenden Artikeln verwendeten flüssigkeitsdurchlässigen Decklagen besteht darin, eine Decklage aus einer Kunststoffolie oder einem Vliesstoff herzustellen und mit Flüssigkeitskanälen zu versehen, die sich jeweils von ihrer Oberseite zu ihrer Unterseite erstrecken, wobei eine obere Oberfläche der Lage für eine Berührung mit der Haut des Trägers eingerichtet ist und die unteren Öffnungen der jeweiligen Flüssigkeitskanäle so angeordnet sind, daß sie in Kontakt mit einem absorbierenden Kern stehen, so daß eine innerhalb jedes Flüssigkeitskanals herrschende Kapillarwirkung dazu dienen kann, ausgeschiedene Körperflüssigkeiten zum absorbierenden Kern zu transportieren.

Beispielsweise beschreibt die Japanische Patent-Offenlegungsschrift Nr. 1985-17081 ein Verfahren, demgemäß eine aus einer Polyethylenfolie bestehende Decklage mit konisch zulaufenden Flüssigkeitskanälen versehen ist und die unteren Enden der jeweiligen Kanäle so angeordnet sind, daß sie in engem Kontakt mit einem absorbierenden Kern stehen.

Entsprechend einer durch Japanese patent application Disclosure Gazette No. 1985-259261 beschriebenen Technik ist eine aus einer Kunststoffolie hergestellte Decklage mit zylindrischen Flüssigkeitskanälen versehen, deren untere Enden sich an ihrer einen Seite in einen absorbierenden Kern hinein erstrecken. Mit der unteren Oberfläche der Decklage ist eine Faserschicht verschweißt, deren Fasern in die Flüssigkeitskanäle an deren anderen Seite hinein ragen. Solche zylindrischen Flüssigkeitskanäle sind stabiler und deshalb unter Einwirkung derselben Last weniger verformbar als die konischen Flüssigkeitskanäle. Zusätzlich werden die zylindrischen Flüssigkeitskanäle den konischen Flüssigkeitskanälen insofern vorgezogen, als daß die sich in die Flüssigkeitskanäle hinein erstreckenden Fasern ebenfalls durch die Kapillarwirkung zur schnelleren Abfuhr der Körperflüssigkeiten zum absorbierenden Kern beitragen.

Bei einer in Japanese patent application Disclosure Gazette No. 1992-152945 beschriebenen Technik wird eine hoch verdichtete Fläche oder eine Versteifungsrippe vorgesehen, die die unteren Öffnungen jeweiliger Flüssigkeitskanäle kontinuierlich umgibt. Dieses Verfahren stabilisiert die Öffnung jedes Flüssigkeitskanals gegen Verformung und auch gegen ein Zusammenfallen sicher und ermöglicht es, daß die Körperflüssigkeiten wirksam zu einem absorbierenden Kern abgeleitet werden.

Für die Decklage ist eine weiche Berührung notwendig, weshalb solche herkömmlichen Decklagen, wie sie die oben erwähnten Patentoffenlegungsschriften und Disclosure Gazettes beschreiben, auch aus dünnem und weichem Material hergestellt sind. Jedoch haben die in Japanese patent application Disclosure Gazette No. 1982-17081 offenbarten, konischen Flüssigkeitskanäle und auch die in Japanese patent application Disclosure Gazette No. 1985-259261 beschriebenen, zylindrischen Flüssigkeitskanäle einen Nachteil mit negativer Folge dahingehend, daß die unteren Öffnungen, d.h. die freien Enden dieser Flüssigkeitskanäle, leicht verformt werden können, so daß die Flüssigkeitskanäle, beispielsweise durch das Körpergewicht eines Trägers, leicht zusammenfallen und dadurch häufig die gleichförmige Abfuhr der Körperflüssigkeit zum absorbierenden Kern behindert wird. Während die Stabilität jedes Flüssigkeitskanals gegen eine axial gegen ihn ausgerichtete Druckkraft mehr oder weniger gesteigert werden kann, fällt ein Flüssigkeitskanal dennoch durch eine quer auf ihn einwirkende Kraft zusammen, da er schließlich weiches Material aufweist. Während die durch obengenannte Japanese patent application Disclosure Gazette No. 1992-152945 beschriebene Technik theoretisch die Gefahr, daß ein Flüssigkeitskanal zusammenfallen kann, vermindert, sind zum einen die Dicke der Decklage und zum anderen der Durchmesser der Flüssigkeitskanäle undurchführbar gering, so daß sich die die unteren Öffnungen der jeweiligen Flüssigkeitskanäle kontinuierlich umgebende hochverdichtete Fläche oder Verstärkungsrippe nur schwer erzielen läßt, obwohl dies eines der wichtigsten Merkmale des beschriebenen Verfahrens darstellt.

Angesichts dieser vom Stand der Technik bisher ungelösten Fragen besteht eine wesentliche Aufgabe der Erfindung darin, eine Decklage anzugeben, bei der eine möglicherweise auftretende Verformung der Flüssigkeitskanäle verhindert oder eingeschränkt ist und so die bei den bekannten Decklagen auftretenden Probleme vermieden werden.

### ZUSAMMENFASSUNG DER ERFINDUNG

Die obengenannte Aufgabe wird erfindungsgemäß bei einer Decklage zur Verwendung von körperflüssigkeitsabsorbierenden Artikeln gelöst, wobei diese Decklage mit einer Vielzahl von Flüssigkeitskanälen, die sich durch die Decklage hindurch von einer oberen Öffnung zu einer unteren Öffnung erstrecken, und einem mit der Haut in Berührung stehenden Bereich versehen ist, der ununterbrochen um die oberen Öffnungen der jeweiligen Flüssigkeitskanäle herum ausgebildet ist. Die Lösung ist dadurch gekennzeichnet, daß diese Decklage eine erste Lage, die einen thermoplastischen Film oder Vliesstoff aus thermoplastischen, miteinander verschweißten Fasern, die Flüssigkeitskanäle und den hautberührenden Bereich aufweist, und die Decklage eine zweite unterhalb der ersten Lage liegende Lage aufweist, welche Vliesstoff aus thermoplastischen, miteinander und mit der ersten Lage um die unteren Öffnungen der jeweiligen Flüssigkeitskanäle herum verschweißte Fasern und einzelne Fasern aufweist, die von der zweiten Lage um die unteren Öffnungen herum aufgelockert nach oben in die jeweiligen Flüssigkeitskanäle entlang deren Innenwände so ragen, daß die zweite Lage von der ersten Lage mit Ausnahme der Umgebung der unteren Öffnungen beabstandet ist und dadurch zusammen mit der ersten Lage Hohlräume bildet, die sich in Dickenrichtung der Decklage erstrecken.

Die Decklage weist somit eine erste Lage und eine unterhalb der ersten Lage liegende zweite Lage auf, wobei die erste Lage mit Flüssigkeitskanälen versehen ist und die zweite Lage mit der ersten Lage um die unteren Öffnungen der jeweiligen Flüssigkeitskanäle herum verschweißt ist.

Vorzugsweise ist die zweite Lage im Vergleich mit der ersten Lage hydrophil.

Eine solche Decklage wird beispielsweise durch Blasen geschmolzener Fasern von einem Extruder gegen die Unterseite der mit den Flüssigkeitskanälen versehenen ersten Lage ermöglicht, wodurch schmelzgeblasener Vliesstoff ausgebildet wird, so daß die schmelzgeblasenen Fasern mit der ersten Lage um die unteren Öffnungen der jeweiligen Flüssigkeitskanäle herum verschmelzen und zum Schutz dieser Kanäle gegen Deformation in der Nähe ihrer unteren Öffnungen beitragen können. Die erste und die zweite Lage hängen um die oberen und unteren Öffnungen der jeweiligen Flüssigkeitskanäle herum zusammen, so daß die Flüssigkeitskanäle im wesentlichen gegen Verformung und Zusammenfallen unter einer auf sie einwirkenden axialen und auch einer transversalen Kraft verfestigt sind.

Flüssige Ausscheidungen fließen in die Flüssigkeitskanäle, erreichen die jeweiligen unteren Öffnungen, woraufhin die flüssigen Ausscheidungen von dem absorbierenden Kern an den Stellen des Kerns, die mit den jeweiligen unteren Öffnungen in Kontakt sind, aufgesaugt werden, verteilen sich dann über die zweite Lage und werden vom absorbierenden Kern auch über einen in Kontakt mit der zweiten Lage stehenden Bereich absorbiert. Die als Ergebnis der Verteilung benetzte zweite Lage vermittelt dem Träger kein spürbares Nässegefühl, da sie mit der ersten Lage um die jeweiligen unteren Öffnungen verschweißt, jedoch reichlich von der ersten Lage in der verbleibenden Zone beabstandet ist. Andere besondere Ausführungen gemäß der Erfindung sind in den Ansprüchen 3-7 angegeben.

### KURZBESCHREIBUNG DER ZEICHNUNGEN

Die Erfindung wird nachfolgend unter Zugrundelegung eines Ausführungsbeispiels unter Bezugnahme auf die beiliegenden Zeichnungen beschrieben. In den Figuren zeigen:
- Fig. 1: eine perspektivische Darstellung einer Monatsbinde mit teilweise herausgebrochenem Bereich;
- Fig. 2: eine Schnittansicht, die einen Abschnitt einer Decklage in vergrößertem Maßstab darstellt; und
- Fig. 3: schematisch ein ein Decklagenherstellungsverfahren veranschaulichendes Diagramm.

### BESCHREIBUNG DES BEVORZUGTEN AUSFÜHRUNGSBEISPIELS

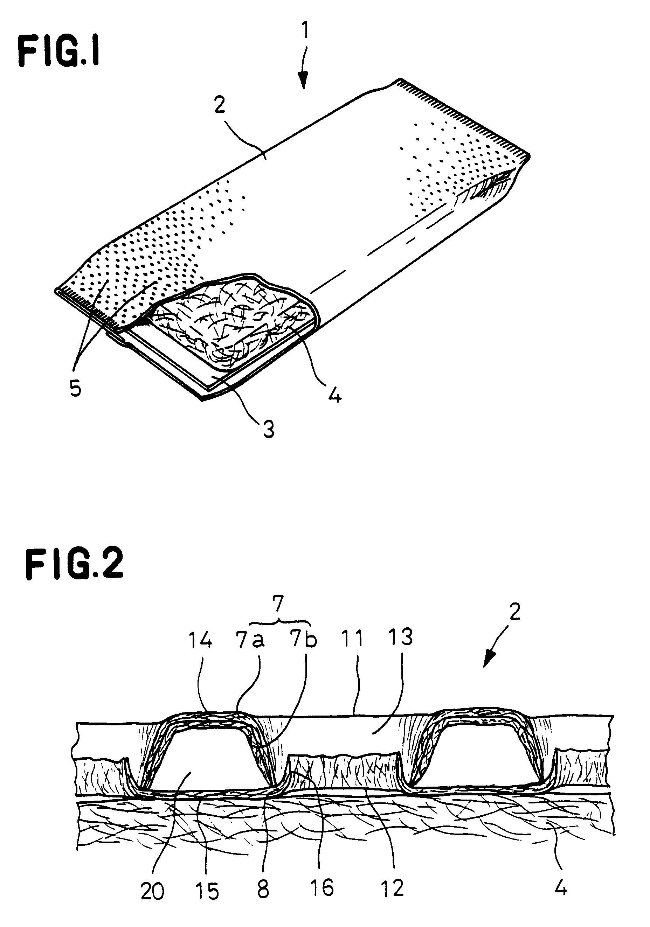

Es wird Bezug genommen auf Fig. 1, die in perspektivischer Darstellung eine Monatsbinde 1 mit teilweise herausgebrochenem Bereich zeigt. Die darin dargestellte Binde 1 weist eine flüssigkeitsdurchlässige Decklage 2, eine flüssigkeitsundurchlässige untere Lage 3 und einen sandwichartig zwischen diese beiden Lagen 2 und 3 eingelegten absorbierenden Kern auf, wobei die Decklage 2 den absorbierenden Kern 4 insgesamt umhüllt und an ihren an der Rückseite der Binde 1 sich überlappenden Seitenteilen und ebenso an ihren an entgegengesetzten Enden des absorbierenden Kerns 4 sich erstreckenden Endabschnitten versiegelt ist. Die untere Lage 3 ist zwischen die auf der Unterseite der Binde 1 liegende Decklage 2 und den absorbierenden Kern 4 gelegt.

Unter Bezug auf Fig. 2, die die Decklage 2 schematisch in einem Teilschnitt in Dickenrichtung veranschaulicht, steht der absorbierende Kern 4 in Kontakt mit der Unterseite der Decklage 2. Die Decklage 2 weist eine obere Lage 7, die ihrerseits ein Laminat aus einer ersten Schicht 7a und einer zweiten Schicht 7b aufweist, die beide aus schmelzgeblasenen Fasern bestehen, und eine untere Lage 8 auf, die unterhalb der oberen Lage 7 liegt und aus schmelzgeblasenen Fasern besteht. Die obere Lage 7 enthält eine Vielzahl von Flüssigkeitskanälen 13, die jeweils durch die Lage 7 von einer oberen Öffnung 11 zu einer unteren Öffnung 12 gehen und einen hautberührenden Bereich 14, der als ununterbrochene, ebene Zone um die jeweiligen oberen Öffnungen 11 herum ausgebildet ist. Die untere Lage 8 enthält einen ebenen Bereich 15, der als ununterbrochene, ebene Zone um die jeweiligen unteren Öffnungen 12 herum gebildet ist und ansteigende Bereiche 16, die vom ebenen Bereich 15 nach oben in die jeweiligen Flüssigkeitskanäle 13 hineinragen. Die die untere Lage 8 bildenden schmelzgeblasenen Fasern sind mit der oberen Lage 7 um die jeweiligen unteren Öffnungen 12 herum verschweißt, und dadurch ist die untere Lage 8 einstückig mit der oberen Lage 7. Jedoch sind Hohlräume 20 zwischen diesen Lagen 7 und 8 gebildet, da der hautberührende Bereich 14 und der ebene Bereich 15 voneinander beabstandet sind.

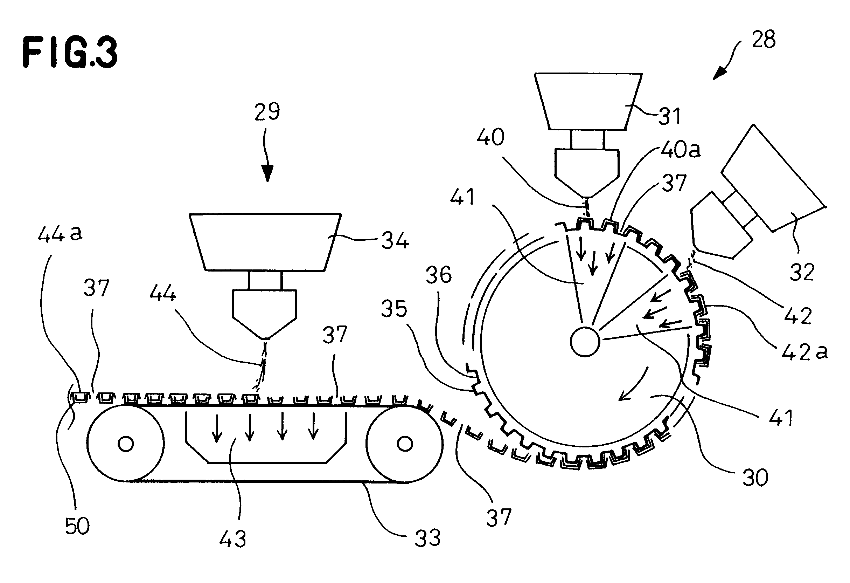

Nun wird Bezug auf Fig. 3 genommen, die schematisch ein Verfahren zur Herstellung der Decklage 2 veranschaulicht. Das Verfahren weist einen ersten Formschritt 28 unter Verwendung einer Formwalze 30 sowie eines ersten und zweiten Schmelzblasextruders 31 und 32, die um die Formwalze 30 herum vorgesehen sind und einen zweiten Formschritt 29 auf, der ein endloses Band 33 und einen dritten Schmelzblasextruder 34 benutzt, die oberhalb des endlosen Bandes 33 angeordnet sind. Die Formwalze 30 ist um ihre äußere periphere Oberfläche herum mit Vorsprüngen 35 und Zahneinschnitten 36 versehen, die abwechselnd angeordnet sind und als Gußformen dienen, so daß schmelzgeblasene Fasern 40 vom ersten Extruder 31 gegen diese Gußformen unter Einwirkung eines Sogs 41 geblasen werden, um eine Vliesschicht 40a zu bilden, die als die erste Schicht 7a der ersten Lage 7 bestimmt ist. In ähnlicher Weise werden schmelzgeblasene Fasern 42 vom zweiten Extruder 32 gegen die Vliesschicht 40a zur Ausbildung einer Vliesschicht 42a geblasen, die als die zweite Schicht 7b der ersten Lage 7 bestimmt ist. Ein Laminat dieser Vliesschichten 40a, 42a wird dann von der Walze 30 zu einem Kontinuum der ersten Lage 7 mit deren hautberührendem Bereich 14 geformt, wie er von den Vorsprüngen 35 und den von den Zahneinschnitten 36 geformten Flüssigkeitskanälen 13 gebildet wird. Es sollte verständlich sein, daß verschiedene Faktoren, wie die jeweils einzublasende Menge der Fasern 40 und 42, die Stärke des jeweiligen Sogs 41 und die Tiefe jedes Zahneinschnitts 36 während des ersten Formschritts 28 zum Erzeugen des Kontinuums der ersten Lage 7 eingestellt werden können, so daß die Lage 7 die Fasern 40 und 42 nach oben längs den Seitenwänden der jeweiligen Zahneinschnitte 36 ragend enthält, um die Flüssigkeitskanäle 13 und die Öffnungen zu bilden, die durch die Böden der jeweiligen Zahnein-schnitte 36 geformt und dazu bestimmt sind, die unteren Öffnungen 12 der ersten Lage 7 zu sein. Das Kontinuum der ersten Lage 7 wird von der peripheren Oberfläche der Formwalze 30 bei seinem Übergang vom ersten Formschritt 28 zum zweiten Formschritt 29 getrennt und auf das endlose Band 33 mit der Oberfläche nach oben abgelegt, die mit der peripheren Oberfläche der Formtrommel 30 in Berührung stand. Durch die Wirkung eines Sogs 43 werden schmelzgeblasene Fasern 44 gegen die nach oben weisende Oberfläche zur Ausbildung einer Vliesschicht 44a geblasen, die als zweite Lage 8 vorgesehen ist. Die Menge der zu blasenden Fasern 44a und die Stärke des Sogs 43 können eingestellt werden, um sicherzustellen, daß die Fasern 44 um die Öffnungen 37 ohne Ausbildung einer ersichtlichen Schicht aus Vliesstoff aufgelockert sind und in die Flüssigkeitskanäle 13 längs deren Innenwände ragen, jedoch auf keinen Fall die als untere Öffnung 12 vorgesehenen Öffnungen 37 ausfüllen. Das Wickel der Lage 50, die diese Vliesstoffschichten 40a, 42a, 44a aufweist, die in der oben beschriebenen Weise erzeugt wurden, kann abgerollt und in Blätter geschnitten werden, die die gewünschten Abmessungen haben, um als einzelne Decklagen 2 verwendet zu werden.

Bei jeder von dem Wickel der Lage 50 abgeschnittenen Decklage 2 sind die die erste oder obere Lage 7 bildenden schmelzgeblasenen Fasern 40, 42 um die unteren Öffnungen 12 in Richtung des Sogs 41 aufgeplustert. Die vom dritten Extruder 34 gegen die obere Lage 7 geblasenen, schmelzgeblasenen Fasern 44 sind mit den anderen schmelzgeblasenen Fasern 40, 42 verschmolzen und/oder verklebt und vereinigen dabei die zweite oder untere Lage 8 mit der oberen Lage 7. Durch die Bildung der oberen Lage 7 mit der zweischichtigen Struktur, die die schmelzgeblasenen Vliesstoffschichten 40a, 42a wie beim veranschaulichten Ausführungsbeispiel aufweist, bei der der schmelzgeblasene Vliesstoff mit höherer Dichte zuerst geformt ist, um die Deckschicht 2 standig zu machen und bei der dann die obere Lage 42a des schmelzgeblasenen Vliesstoffs mit geringerer Dichte geformt wird, lassen sich auf einfache Weise eine solche Decklage 2 verwendende Artikel erzielen, die ihren Trägern eine kleidungsähnliche, weiche Berührung vermitteln. Es sollte verständlich sein, daß die obere Lage 7 auch aus irgendwelchen anderen Vliesstoffschichten 40a, 42a gebildet sein kann.

Die schmelzgeblasenen Fasern 40, 42, 44 können aus irgendeinem geeigneten thermoplastischen Kunstharz, wie Polyethylen, bestehen. Innerhalb des Schutzbereichs der Erfindung besteht auch die Möglichkeit, den schmelzgeblasenen Vliesstoff der oberen Lage 7 durch perforierten Kunststoffilm zu ersetzen. Bevorzugt wird die obere Lage 7 aus geeignetem hydrophoben Material hergestellt, durch das einerseits ein trockenes Gefühl auch nach Ausscheidung der Körperflüssigkeiten erhalten bleibt, und die untere Lage 8 wird im Vergleich mit der oberen Lage 7 hydrophil gemacht, so daß die Körperflüssigkeiten zum absorbierenden Kern übergehen und sich weitgehend über die untere Lage 8 so schnell wie möglich ausbreiten können. Eine solche untere Lage 8 kann beispielsweise unter Verwendung von Polyethylen, das zuvor mit einem hydrophilen Wirkstoff gemischt wurde, erhalten werden.

In der erfindungsgemäß aufgebauten Decklage sind die Flüssigkeitskanäle wirksam formstabilisiert und schwer zu blockieren, da die untere Lage mit der oberen Lage um die unteren Öffnungen der jeweiligen Flüssigkeitskanäle verschweißt ist.

Die über der Decklage ausgeschiedenen Körperflüssigkeiten fließen in die Flüssigkeitskanäle und erreichen deren untere Öffnungen, woraufhin die Körperflüssigkeiten von den Teilen des absorbierenden Kerns, die in Kontakt mit diesen unteren Öffnungen stehen, absorbiert werden und sich gleichzeitig über die untere Lage verteilen können, so daß die Körperflüssigkeiten auch von dem Teil, der sich in Kontakt mit der unteren Lage befindet, absorbiert werden. Auf diese Weise gestattet die Decklage der Erfindung eine Erhöhung der Absorptionsgeschwindigkeit im Vergleich mit den Decklagen des Standes der Technik.

Die untere Lage der Decklage vermittelt kein nasses Gefühl an den Träger, auch wenn die untere Lage mit den darüber sich ausbreitenden Körperflüssigkeiten benetzt wurde, weil die Hohlräume zwischen der oberen und unteren Lage gebildet sind.

Eine sehr gute Formstabilität der Flüssigkeitskanäle verbessern die Polsterwirkung und deshalb den Tragekomfort der absorbierenden Artikel.

## Patentansprüche

1. Decklage (2) zur Verwendung bei körperflüssig-keitsabsorbierenden Artikeln, wobei diese Decklage (2) mit einer Vielzahl von Flüssigkeitskanälen (13), die sich jeweils von einer oberen Öffnung zu einer unteren Öffnung durch die Decklage hindurch erstrecken und mit einem mit der Haut in Berührung stehenden Bereich (14) versehen ist, der ununterbrochen um die oberen Öffnungen (11) der jeweiligen Flüssigkeitskanäle (13) herum ausgebildet ist, dadurch **gekennzeichnet**, daß diese Decklage (2) eine erste Lage (7) aufweist, die ihrerseits einen thermoplastischen Film oder Vliesstoff aus miteinander verschweißten, thermoplastischen Fasern, die Flüssigkeitskanäle (13) und den hautberührenden Bereich (14) aufweist, eine zweite Lage (8) aufweist, die unter der ersten Lage (7) liegt und die Vliesstoff aus thermoplastischen, miteinander und mit der ersten Lage (7) um die unteren Öffnungen (12) der jeweiligen Flüssigkeitskanäle (13) herum verschweißte Fasern und einzelne Fasern (16) aufweist, die sich von der zweiten Lage (8) um die unteren Öffnungen (12) herum aufgelockert nach oben in die jeweiligen Flüssigkeitskanäle (13) entlang deren Innenwände erstrecken, so daß die zweite Lage (8) von der ersten Lage (7) mit Ausnahme der Umgebung um die unteren Öffnungen (12) beabstandet ist und dadurch zusammen mit der ersten Lage (7) Hohlräume (20) bildet, die sich in Dickenrichtung der Decklage (2) erstrecken.

2. Decklage nach Anspruch 1, wobei die zweite Lage (8) im Vergleich mit der ersten Lage (7) hydrophil ist.

3. Decklage nach Anspruch 1 oder 2, wobei die erste Lage (7) perforierten Polyethylenfilm und die zweite Lage schmelzgeblasen Vliesstoff (42a) aus Polyethylen aufweisen.

4. Decklage nach Anspruch 1 oder 2, wobei die erste Lage (7) und die zweite Lage (8) schmelzgeblasenen Vliesstoff aus Polyethylen aufweisen.

5. Decklage nach einem der Ansprüche 1 bis 4, wobei die zweite Lage (8) schmelzgeblasenen Vliesstoff aus Polyethylen aufweist, das zuvor mit hydrophilem Wirkstoff gemischt wurde.

6. Decklage nach Anspruch 1, wobei die erste Lage (7) eine obere und untere Schicht (7a, 7b) aus Vliesstoff aufweist, von denen die obere Schicht (7a) eine Dichte hat, die kleiner ist als die der unteren Schicht (7b).

7. Decklage nach Anspruch 6, wobei die obere und die untere Schicht (7a, 7b) schmelzgeblasenen Vliesstoff aufweisen.

## Claims

1. Top sheet (2) for use in disposable articles which absorb body fluids, this top sheet (2) being provided with a large number of fluid channels (13), which each extend through the top sheet from an upper opening to a lower opening, and with a region (14) which is in contact with the skin and which is designed to be continuous around the upper openings (11) of the respective fluid channels (13),
**characterised in that**
this top sheet (2) has a first layer (7), which itself has a thermoplastic film or nonwoven fabric made of thermoplastic threads welded together, the fluid channels (13) and the region (14) touching the skin, and a second layer (8) which lies below the first layer (7) and which has nonwoven fabric made of thermoplastic threads welded together with one another and with the first layer (7) around the lower openings (12) of the respective fluid channels (13) and individual threads (16) which extend from the second layer (8) around the lower openings (12), loosened, upwards into the respective fluid channels (13), along the inner walls of same, so that the second layer (8) is at a distance from the first layer (7) with the exception of the part surrounding the lower openings (12) and thus forms, together with the first layer (7), hollow spaces (20) which extend in the direction of the thickness of the top sheet (2).

2. Top sheet according to claim 1, in which the second layer (8) is hydrophilic in comparison with the first layer (7).

3. Top sheet according to claim 1 or 2, in which the first layer (7) has perforated polyethylene film and the second layer has fusion-blown nonwoven fabric (42a) made of polyethylene.

4. Top sheet according to claim 1 or 2, in which the first layer (7) and the second layer (8) has fusion-blown nonwoven fabric made of polyethylene.

5. Top sheet according to one of claims 1 to 4, in which the second layer (8) has fusion-blown nonwoven fabric made of polyethylene which has been previously mixed with a hydrophilic agent.

6. Top sheet according to claim 1, in which the first layer (7) has an upper and a lower layer (7a, 7b) made of nonwoven fabric, the upper (7a) of which two layers has a thickness which is smaller than that of the lower layer (7b).

7. Top sheet according to claim 6, in which the upper and the lower layers (7a, 7b) have fusion-blown nonwoven fabric.

## Revendications

1. Couche de voilage (2) pour utilisation avec des articles absorbant les fluides corporels, ladite couche de voilage (2) étant pourvue d'une multitude de passages à liquides (13) s'étendant, respectivement, d'une ouverture supérieure vers une ouverture inférieure, en traversant la couche de voilage, et d'une zone (14) en contact avec la peau, formée, sans discontinuité, autour des ouvertures supérieures (11) des passages à liquides (13), caractérisée en ce que cette couche de voilage (2) présente une première couche (7), comportant une pellicule thermoplastique ou un non tissé de fibres thermoplastiques soudées entre elles, les passages à liquide (13) et la zone (14) en contact avec la peau ; une seconde couche (8) sous-jacente à la première (7) et présentant un non tissé de fibres thermoplastiques soudées entre elles et avec la première couche (7), autour des ouvertures inférieures (12) des passages à liquide (13), et des fibres individuelles (16) qui s'étendent et se démêlant à partir de la seconde couche (8) sur le pourtour des ouvertures inférieures (12), vers le haut, dans les passages à liquide (13), le long de leurs parois intérieures, de sorte que la seconde couche (8) est écartée de la première couche (7), exception faite des parties entourant les ouvertures inférieures (12) et donne ainsi naissance, conjointement avec la première couche (7), à des cavités (20) qui s'étendent dans le sens de l'épaisseur de la couche de voilage (2).

2. Couche de voilage selon la revendication 1, dans laquelle la seconde couche (8) est hydrophile par comparaison à la première couche (7).

3. Couche de voilage selon l'une des revendications 1 ou 2, dans laquelle la première couche (7) comprend une pellicule de polyéthylène perforée, et la seconde, un non tissé de polyéthylène soufflé en fusion (42a).

4. Couche de voilage selon l'une des revendications 1 ou 2, dans laquelle la première couche (7) et la seconde (8) comprennent un non tissé de polyéthylène soufflé en fusion.

5. Couche de voilage selon l'une quelconque des revendications 1 à 4, dans laquelle la seconde couche (8) présente un non tissé de polyéthylène soufflé en fusion, préalablement mélangé à de la matière active hydrophile.

6. Couche de voilage selon la revendication 1, dans laquelle la première couche (7) comporte une couche supérieure et une couche inférieure (7a, 7b) de non tissé, dont la couche supérieure (7a) présente une densité inférieure à celle de la couche inférieure (7b).

7. Couche de voilage selon la revendication 6, dans laquelle les couches supérieure et inférieure (7a, 7b) présentent un non tissé soufflé en fusion.
